# EUROPEAN PATENT APPLICATION

(11) **EP 4 810 119 A2**
(43) Date of publication of application: **23.09.2026**
(21) Application number: 26176531.7
(22) Date of filing: 28.01.2021
(51) Int. Cl.: A61P 35/00

(54) **COMBINATION THERAPY FOR TREATING ABNORMAL CELL GROWTH**

(30) Priority: 31.01.2020 US 202062968615 P; 18.11.2020 US 202063115433 P
(62) Document number(s) of the earlier application(s) in accordance with Art. 76 EPC: 21747362.8 / 4 096 671
(71) Applicant: Verastem, Inc., Needham, MA 02494 (US)
(72) Inventor: PACHTER, Jonathan A., Wayland, MA 01778 (US); COMA, Silvia, Needham, MA 02494 (US)
(74) Representative: Heller, Benjamin Henry

(57) **Abstract**

The present invention relates to methods, compositions, and oral dosage forms of a KRAS G12C inhibitor in combination with a FAK inhibitor and/or a MEK inhibitor or a dual RAF/MEK inhibitor, for treating abnormal cell growth (e.g., cancer).

## Description

### CROSS REFERENCE TO RELATED APPLICATIONS

This application claims priority to and the benefit of U.S. Provisional Patent Application Number 62/968,615, filed January 31, 2020, and 63/115,433, filed November 18, 2020, each of which is incorporated herein by reference in its entirety.

### BACKGROUND

Convincing evidence suggests that Focal Adhesion Kinase (FAK), a cytoplasmic, non-receptor tyrosine kinase, plays an essential role in cell survival, proliferation, migration, invasion, and adhesion (Clark and Brugge 1995, Science 268: 233-239) and its aberrant activation is associated with an increase in the metastatic potential of tumors (Owens et al. 1995, Cancer Research 55: 2752-2755). Selective inhibitors of certain non-receptor tyrosine kinases, such as FAK, ICK, SRC, ABL or serine/threonine kinases (e.g., cyclin dependent kinases), are useful in the treatment of abnormal cell growth, in particular cancer, in mammals. FAK is also known as the Protein-Tyrosine Kinase 2, PTK2. FAK expression and/or activity has been reported to be upregulated in a range of malignancies, including uveal melanoma and cancers of the thyroid, prostate, cervix, colon, rectum, oral epithelium, ovary, and breast.

Kirsten Rat Sarcoma 2 Viral Oncogene Homolog (KRAS) is a small GTPase and a member of the Ras family of oncogenes. KRAS serves as a molecular switch cycling between inactive (GDP-bound) and active (GTP-bound) states to transduce upstream cellular signals received from multiple tyrosine kinases to downstream effectors to regulate a wide variety of processes, including cellular proliferation (e.g., see Alamgeer et al., (2013) Current Opin Pharmcol. 13:394-401). KRAS gene mutations are common in cancer, for example, pancreatic cancer, lung adenocarcinoma, colorectal cancer (CRC), gall bladder cancer, thyroid cancer, and bile duct cancer (Kodaz et al., EJMO 2017). KRAS mutations are observed in about 30% of patients with lung adenocarcinoma, 40% of patients with CRC and 67% of patients with pancreatic adenocarcinomas. Whereas KRAS is a major driver in lung adenocarcinomas and in pancreatic cancers, KRAS is not a primary initiating event in colorectal cancer (McCormick, 2015).

Components of the RAS/RAF/MEK/ERK signal transduction pathway also represent opportunities for the treatment of abnormal cell growth, e.g., cancer. Selective inhibitors of certain components of the RAS/RAF/MEK/ERK signal transduction pathway, such as RAS, RAF, MEK and ERK, are useful in the treatment of abnormal cell growth, in particular cancer, in maminals.

Due to the severity and breadth of diseases and disorders associated with abnormal cell growth (e.g., cancer), there is a need for effective therapeutic means and methods for treatment. The compounds, compound combinations, compositions, and methods described herein are directed toward this end.

### SUMMARY

Provided herein, in part, are combinations (e.g., combinations of compounds as described herein, e.g., KRAS G12C inhibitor in combination with a FAK inhibitor and/or a MEK inhibitor or a dual RAF/MEK inhibitor), which can be used, for example, in methods of treating abnormal cell growth (e.g., cancer) in a subject in need thereof.

Thus, in an aspect, provided herein is a method of treating a cancer in a subject in need thereof, the method comprising administering to the subject a KRAS G12C inhibitor (e.g., ARS-853, ARS-1620, ARS-3248, LY3499446, AMG-510, and MRTX849) or a pharmaceutically acceptable salt thereof in combination with a FAK inhibitor (e.g., defactinib, TAE226, BI-853520 (IN10018), GSK2256098, PF-03814735, BI-4464, VS-4718, and APG-2449) or a pharmaceutically acceptable salt thereof, thereby treating the subject.

In another aspect, provided herein is a method of treating a cancer in a subject in need thereof, the method comprising administering to the subject a KRAS G12C inhibitor (e.g., ARS-853, ARS-1620, ARS-3248, LY3499446, AMG-510, and MRTX849) or a pharmaceutically acceptable salt thereof in combination with a MEK inhibitor (e.g., trametinib, cobimetinib, binimetinib, selumetinib, PD-325901, CI-1040, CH5126766, MEK162, AZD8330, GDC-0623, refametinib, pimasertib, WX-554, HL-085, CH4987655, TAK-733, CInQ-03, G-573, PD184161, PD318088, PD98059, RO5068760, U0126, and SL327, e.g., CH5126766) or a pharmaceutically acceptable salt thereof, thereby treating the subject.

In one aspect, disclosed herein is a method of treating a cancer in a subject in need thereof, the method comprising administering to the subject a KRAS G12C inhibitor (e.g., ARS-853, ARS-1620, ARS-3248, LY3499446, AMG-510, and MRTX849) or a pharmaceutically acceptable salt thereof in combination with a dual RAF/MEK inhibitor (e.g., CH5126766) or a pharmaceutically acceptable salt thereof, thereby treating the subjec

Also provided herein is a method of treating a cancer in a subject in need thereof, the method comprising administering to the subject a KRAS G12C inhibitor (e.g., ARS-853, ARS-1620, ARS-3248, LY3499446, AMG-510, and MRTX849) or a pharmaceutically acceptable salt thereof in combination with a FAK inhibitor (e.g., defactinib, TAE226, BI-853520 (IN10018), GSK2256098, PF-03814735, BI-4464, VS-4718, and APG-2449) or a pharmaceutically acceptable salt thereof and a MEK inhibitor (e.g., trametinib, cobimetinib, binimetinib, selumetinib, PD-325901, CI-1040, CH5126766, MEK162, AZD8330, GDC-0623, refametinib, pimasertib, WX-554, HL-085, CH4987655, TAK-733, CInQ-03, G-573, PD184161, PD318088, PD98059, RO5068760, U0126, and SL327) or a pharmaceutically acceptable salt thereof, thereby treating the subject.

In some embodiments, the KRAS G12C inhibitor is selected from the group consisting of ARS-853, ARS-1620, ARS-3248, LY3499446, AMG-510, and MRTX849, or a pharmaceutically acceptable salt thereof. In some embodiments, the KRAS G12C inhibitor is AMG-510 or a pharmaceutically acceptable salt thereof. In some embodiments, the KRAS G12C inhibitor is MRTX849 or a pharmaceutically acceptable salt thereof.

In some embodiments, the FAK inhibitor is selected from the group consisting of defactinib, TAE226, BI-853520 (IN10018), GSK2256098, PF-03814735, BI-4464, VS-4718, and APG-2449, or a pharmaceutically acceptable salt thereof. In some embodiments, the FAK inhibitor is defactinib or a pharmaceutically acceptable salt thereof.

In some embodiments, the method further comprises administering a MEK inhibitor.

In some embodiments, the MEK inhibitor is selected from the group consisting of trametinib, cobimetinib, binimetinib, selumetinib, PD-325901, CI-1040, CH5126766, MEK162, AZD8330, GDC-0623, refametinib, pimasertib, WX-554, HL-085, CH4987655, TAK-733, CInQ-03, G-573, PD184161, PD318088, PD98059, RO5068760, U0126, and SL327, or a pharmaceutically acceptable salt thereof.

In some embodiments, the MEK inhibitor is a dual RAF/MEK inhibitor.

In some embodiments, the MEK inhibitor is CH5126766 or a pharmaceutically acceptable salt thereof.

In some embodiments, the KRAS G12C inhibitor is dosed at about 100 mg to about 2000 mg. In some embodiments, the KRAS G12C inhibitor is administered once daily. In some embodiments, the KRAS G12C inhibitor is administered twice daily. In some embodiments, the KRAS G12C inhibitor is administered orally.

In some embodiments, the FAK inhibitor (e.g., defactinib) is dosed twice daily. In some embodiments, the FAK inhibitor (e.g., defactinib) is dosed once daily. In some embodiments, the FAK inhibitor (e.g., defactinib) is dosed at about 100 mg to about 1000 mg. In some embodiments, the FAK inhibitor (e.g., defactinib) is dosed at about 200 mg to about 400 mg. In some embodiments, the FAK inhibitor (e.g., defactinib) is administered orally.

In some embodiments, the FAK inhibitor is administered before the KRAS G12C inhibitor is administered. In some embodiments, the FAK inhibitor is administered after the KRAS G12C inhibitor is administered. In some embodiments, the FAK inhibitor is administered concurrently with the KRAS G12C inhibitor.

In some embodiments, the MEK inhibitor is dosed at least once a week (e.g., once a week, twice a week, three times a week, four times a week, five times a week, or six times a week). In some embodiments, the MEK inhibitor is dosed once a week. In some embodiments, the MEK inhibitor is dosed twice a week. In some embodiments, the MEK inhibitor is dosed once daily. In some embodiments, the MEK inhibitor is dosed twice daily. In some embodiments, the MEK inhibitor is dosed at about 0.1 mg to about 100 mg. In some embodiments, the MEK inhibitor is administered orally.

In some embodiments, the MEK inhibitor is a dual RAF/MEK inhibitor. In some embodiments, the dual RAF/MEK inhibitor is administered before the KRAS G12C inhibitor is administered. In some embodiments, the dual RAF/MEK inhibitor is administered after the KRAS G12C inhibitor is administered. In some embodiments, the dual RAF/MEK inhibitor is administered concurrently with the KRAS G12C inhibitor.

In some embodiments, the cancer is a cancer with a KRAS G12C mutation.

In some embodiments, the cancer is lung adenocarcinoma, non-small cell lung carcinoma, colorectal cancer (CRC), uterine endometrioid carcinoma, bladder urothelial carcinoma, breast invasive lobular carcinoma, cervical squamous cell carcinoma, cutaneous melanoma, endocervical adenocarcinoma, hepatocellular carcinoma, pancreatic adenocarcinoma, biphasic type pleural mesothelioma, renal clear cell carcinoma, renal clear cell carcinoma, stomach adenocarcinoma, tubular stomach adenocarcinoma, uterine carcinosarcoma, or uterine malignant mixed Mullerian tumor.

Other objects and advantages will become apparent to those skilled in the art from a consideration of the ensuing Detailed Description, Examples, and Claims.

### BRIEF DESCRIPTION OF THE DRAWINGS

**FIG. 1A** shows the synergy score (Loewe model) between VS-6766 and AMG-510 or MRTX849 in KRAS G12C mutant NSCLC and CRC cell lines.
**FIG. 1B** shows the dose response of AMG-510 or MRTX849 alone or combined with VS-6766 (left) and VS-6766 alone or combined with AMG-510 or MRTX849 (right) on tumor cell line viability in H2122 KRAS G12C mutant NSCLC.
**FIG. 2** shows the heatmaps showing synergy scores (Loewe model) between VS-6766 and AMG-510 or MRTX849 (left) and Defactinib and AMG-510 or MRTX849 (right) in KRAS G12C mutant NSCLC and CRC cell lines.
**FIG. 3** shows immunoblot protein western analyses of pERK in KRAS G12C mt NSCLC cell lines treated for 4 and 48 hours with 100 nM VS-6766 and 100 nM AMG-510 or MRTX849.
**FIG. 4** shows immunoblot protein western analyses of KRAS pathway targets (pMEK, pERK and p-p90RSK) in KRAS G12C mt NSCLC cell lines treated for 4 and 48 hours with 100 nM VS-6766 and 100 nM AMG-510 or MRTX849.
**FIG. 5** shows immunoblot protein western analyses of KRAS pathway targets (pMEK and pERK) and quantification of pMEK and pERK by western blot densitometry.
**FIG. 6** shows change in tumor volumes in H2122 tumor bearing mice treated with VS-6766 +/- FAKi +/- AMG-510 for 10 days.

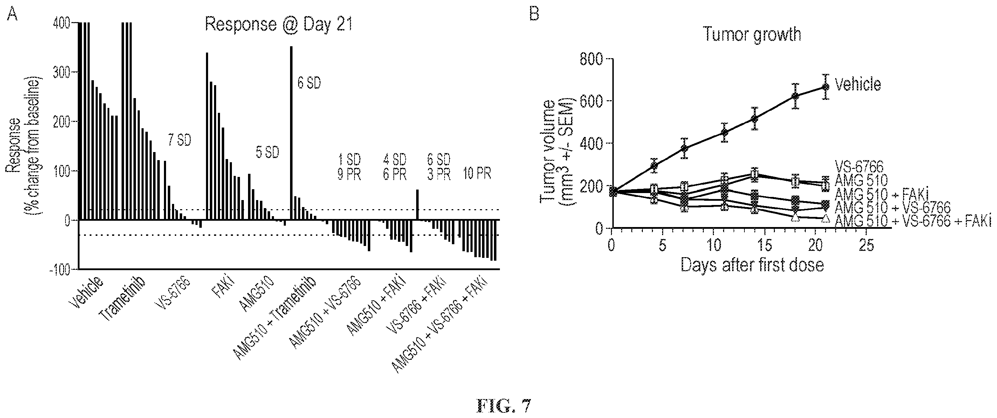

**FIG. 7** shows change in tumor volumes in H358 tumor bearing mice treated with VS-6766 +/-FAKi +/- AMG-510 for 21 days.

### DETAILED DESCRIPTION

As generally described herein, the present disclosure provides methods and combinations of compounds useful for treating abnormal cell growth (e.g., cancer) in a subject in need thereof.

### Definitions

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

As used herein, "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describes pharmaceutically acceptable salts in detail in J. Pharmaceutical Sciences (1977) 66:1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

As used herein, "pharmaceutically acceptable carrier" refers to a non-toxic carrier, adjuvant, or vehicle that does not destroy the pharmacological activity of the compound with which it is formulated. Pharmaceutically acceptable carriers, adjuvants or vehicles that may be used in the compositions described herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

As used herein, a "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition (also "therapeutic treatment").

In general, the "effective amount" of a compound refers to an amount sufficient to elicit the desired biological response. As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, "prophylactic treatment" contemplates an action that occurs before a subject begins to suffer from the specified disease, disorder or condition.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

The term, "oral dosage form," as used herein, refers to a composition or medium used to administer an agent to a subject. Typically, an oral dosage form is administered via the mouth, however, "oral dosage form" is intended to cover any substance which is administered to a subject and is absorbed across a membrane, e.g., a mucosal membrane, of the gastrointestinal tract, including, e.g., the mouth, esophagus, stomach, small intestine, large intestine, and colon. For example, "oral dosage form" covers a solution which is administered through a feeding tube into the stomach.

### Methods of Treatment

Combinations of compounds described herein (e.g., a KRAS G12C inhibitor in combination with a FAK inhibitor and/or a MEK inhibitor or a dual RAF/MEK inhibitor) and pharmaceutical compositions thereof are generally useful in methods of treating abnormal cell growth such as cancer.

Thus, in an aspect, provided herein is a method of treating a cancer in a subject in need thereof, the method comprising administering to the subject a KRAS G12C inhibitor or a pharmaceutically acceptable salt thereof in combination with a FAK inhibitor or a pharmaceutically acceptable salt thereof, thereby treating the subject. In some embodiments, the method further comprises administering a MEK inhibitor.

In another aspect, provided herein is a method of treating a cancer in a subject in need thereof, the method comprising administering to the subject a KRAS G12C inhibitor or a pharmaceutically acceptable salt thereof in combination with a MEK inhibitor or a pharmaceutically acceptable salt thereof, thereby treating the subject.

In one aspect, disclosed herein is a method of treating a cancer in a subject in need thereof, the method comprising administering to the subject a KRAS G12C inhibitor or a pharmaceutically acceptable salt thereof in combination with a dual RAF/MEK inhibitor (or a pharmaceutically acceptable salt thereof, thereby treating the subject.

Also provided herein is a method of treating a cancer in a subject in need thereof, the method comprising administering to the subject a KRAS G12C inhibitor or a pharmaceutically acceptable salt thereof in combination with a FAK inhibitor or a pharmaceutically acceptable salt thereof and a MEK inhibitor or a pharmaceutically acceptable salt thereof, thereby treating the subject.

In some embodiments of the methods described herein, the duration of response to KRAS G12C inhibitor may be reduced by administering to a subject in need thereof a KRAS G12C inhibitor in combination with a FAK inhibitor and/or MEK inhibitor. In some embodiments, the duration of response to KRAS G12C inhibitor may be reduced by administering to a subject in need thereof a KRAS G12C inhibitor in combination with a FAK inhibitor and/or dual RAF/MEK inhibitor. In some embodiments, the combinations as described herein may improve the depth and/or duration of the response (e.g., antitumor response) in the subject.

A contemplated subject for the methods described herein may be identified (e.g., by screening, e.g., sequencing) as having a KRAS G12C mutation.

### KRAS G12C inhibitors

Exemplary KRAS G12C inhibitors include, but are not limited to:
MRTX849 (adagrasib) having the following structure: 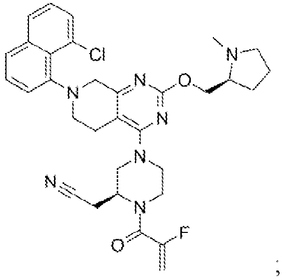
AMG-510 (sotorasib) having the following structure: 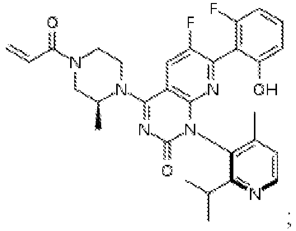
ARS-1620 having the following structure: 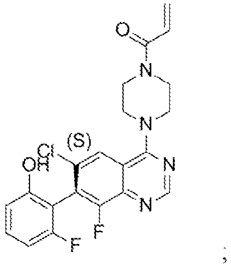
ARS-853 having the following structure: 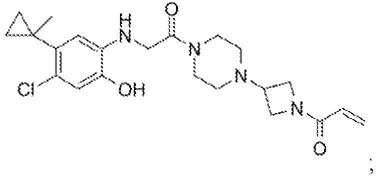
LY3499446 (Eli Lilly); and
ARS-3248 (Araxes Pharma/Wellspring Biosciences).

In some embodiments, the KRAS G12C inhibitor is selected from the group consisting of ARS-853, ARS-1620, ARS-3248, LY3499446, AMG-510, and MRTX849, or a pharmaceutically acceptable salt thereof. In some embodiments, the KRAS G12C inhibitor is AMG-510 or a pharmaceutically acceptable salt thereof. In some embodiments, the KRAS G12C inhibitor is MRTX849 or a pharmaceutically acceptable salt thereof.

In some embodiments, the KRAS G12C inhibitor is administered at least once daily. In some embodiments, the KRAS G12C inhibitor is administered once daily. In some embodiments, the KRAS G12C inhibitor is administered twice daily. In some embodiments, the KRAS G12C inhibitor is administered orally.

In some embodiments, the KRAS G12C inhibitor is dosed at about 10 mg to about 2000 mg, e.g., about 100 mg to about 2000 mg, about 100 mg to about 1500 mg, about 100 mg to about 1000 mg, about 100 mg to about 800 mg, about 100 mg to about 600 mg, about 100 mg to about 400 mg, about 100 mg to about 200 mg, about 200 mg to about 2000 mg, about 200 mg to about 1500 mg, about 200 mg to about 1000 mg, about 200 mg to about 800 mg, about 200 mg to about 600 mg, about 200 mg to about 400 mg, about 400 mg to about 2000 mg, about 400 mg to about 1500 mg, about 400 mg to about 1000 mg, about 400 mg to about 800 mg, about 400 mg to about 600 mg, about 600 mg to about 2000 mg, about 600 mg to about 1500 mg, about 600 mg to about 1000 mg, about 600 mg to about 800 mg, about 800 mg to about 2000 mg, 800 mg to about 1500 mg, about 800 mg to about 1000 mg, about 600 mg to about 2000 mg, about 600 mg to about 1500 mg, about 600 mg to about 1000 mg, about 600 mg to about 800 mg. In some embodiments, the KRAS G12C inhibitor is dosed at about 100 mg. In some embodiments, the KRAS G12C inhibitor is dosed at about 200 mg. In some embodiments, the KRAS G12C inhibitor is dosed at about 300 mg. In some embodiments, the KRAS G12C inhibitor is dosed at about 400 mg. In some embodiments, the KRAS G12C inhibitor is dosed at about 500 mg. In some embodiments, the KRAS G12C inhibitor is dosed at about 600 mg. In some embodiments, the KRAS G12C inhibitor is dosed at about 700 mg. In some embodiments, the KRAS G12C inhibitor is dosed at about 800 mg. In some embodiments, the KRAS G12C inhibitor is dosed at about 900 mg. In some embodiments, the KRAS G12C inhibitor is dosed at about 1000 mg.

### FAK Inhibitors

Potent inhibitors of the FAK protein tyrosine kinases may be adapted to therapeutic use as antiproliferative agents (e.g., anticancer), antitumor (e.g., effective against solid tumors), antiangiogenesis (e.g., stop or prevent proliferation of blood vessels) in mammals, particularly in humans. The compounds described herein, e.g., FAK inhibitors, may be useful in the prevention and treatment of a disease or disorder described herein (e.g., abnormal cell growth, e.g., cancer (e.g., a cancer described herein)). The compounds described herein, e.g., FAK inhibitors, may be useful in the prevention and treatment of non-hematologic malignancies, a variety of human hyperproliferative disorders such as malignant and benign tumors of the liver, kidney, bladder, breast, gastric, ovarian, colorectal, prostate, pancreatic, lung, vulval, thyroid, hepatic carcinomas, sarcomas, glioblastomas, head and neck, and other hyperplastic conditions such as benign hyperplasia of the skin (e.g., psoriasis) and benign hyperplasia of the prostate (e.g., BPH), and in the prevention and treatment of disorders such as mesothelioma. In some embodiments, the compounds described herein, e.g., FAK inhibitors, inhibit protein tyrosine kinase 2 (PYK2).

An exemplary FAK inhibitor includes, but is not limited to, defactinib having the following structure: 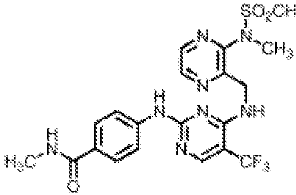 or a pharmaceutically acceptable salt thereof. Defactinib is also known as VS-6063 (e.g., VS-6063 free base) or PF-04554878. VS-6063 and related compounds are also disclosed in, for example, U.S. Patent No. 7,928,109, the content of which is incorporated herein by reference. In some embodiments, VS-6063 can form a pharmaceutically acceptable salt (e.g., VS-6063 hydrochloride).

In some embodiments, the FAK inhibitor is VS-4718, having the following structure: 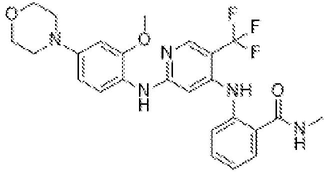 or a pharmaceutically acceptable salt thereof.

In some embodiments, the FAK inhibitor is TAE226, having the following structure: 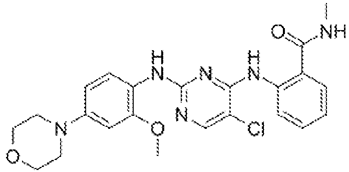 or a pharmaceutically acceptable salt thereof.

In some embodiments, the FAK inhibitor is GSK2256098, having the following structure: 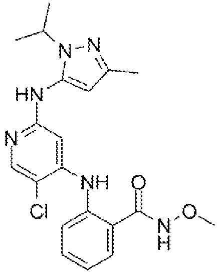 or a pharmaceutically acceptable salt thereof.

In some embodiments, the FAK inhibitor is PF-03814735, having the following structure: 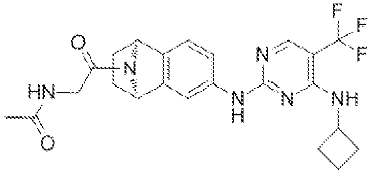 or a pharmaceutically acceptable salt thereof.

In some embodiments, the FAK inhibitor is BI-4464, having the following structure: 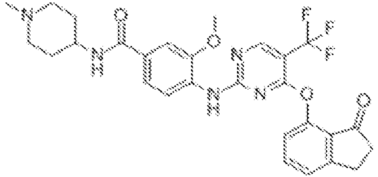 or a pharmaceutically acceptable salt thereof.

In some embodiments, the FAK inhibitor is BI-853520 (IN10018; Boehringer Ingelheim). In some other embodiments, the FAK inhibitor is APG-2449 (Ascentage Pharma Group).

In some embodiments, the FAK inhibitor is selected from the group consisting of defactinib, TAE226, BI-853520, GSK2256098, PF-03814735, BI-4464, VS-4718, and APG-2449, or a pharmaceutically acceptable salt thereof. For example, the FAK inhibitor is defactinib or a pharmaceutically acceptable salt thereof.

In some embodiments, the FAK inhibitor (e.g., defactinib) is dosed at least once daily. For example, in some embodiments, the FAK inhibitor (e.g., defactinib) is dosed twice daily. In some embodiments, the FAK inhibitor (e.g., defactinib) is dosed once daily.

In some embodiments, the FAK inhibitor (e.g., defactinib) is dosed at about 100 mg to about 1000 mg, e.g., about 100 mg to about 800 mg, about 100 mg to about 600 mg, about 100 mg to about 400 mg, about 100 mg to about 200 mg, about 200 mg to about 1000 mg, about 400 mg to about 1000 mg, about 600 mg to about 1000 mg, about 800 mg to about 1000 mg, about 200 mg to about 800 mg, about 200 mg to about 600 mg, about 200 mg to about 400 mg, about 400 mg to about 800 mg, or about 400 mg to about 600 mg. In some embodiments, the FAK inhibitor (e.g., defactinib) is dosed at about 200 mg to about 400 mg. In some embodiments, the FAK inhibitor (e.g., defactinib) is dosed at about 100 mg. In some embodiments, the FAK inhibitor (e.g., defactinib) is dosed at about 200 mg. In some embodiments, the FAK inhibitor (e.g., defactinib) is dosed at about 300 mg. In some embodiments, the FAK inhibitor (e.g., defactinib) is dosed at about 400 mg. In some embodiments, the FAK inhibitor (e.g., defactinib) is dosed at about 500 mg. In some embodiments, the FAK inhibitor (e.g., defactinib) is dosed at about 600 mg. In some embodiments, the FAK inhibitor (e.g., defactinib) is administered orally.

### MEK Inhibitors

A MEK inhibitor can be a small molecule or biologic inhibitor of the mitogen-activated protein kinase (MAPK) enzymes MEK1 and/or MEK2 (e.g., MAPK/ERK pathway).

Examples of MEK inhibitors include, but are not limited to, trametinib (also known as Mekinst, GSK1120212) having the following structure: 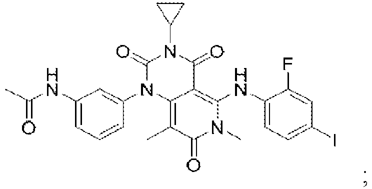 Cobimetinib (also known as GDC-0973, XL518) having the following structure: 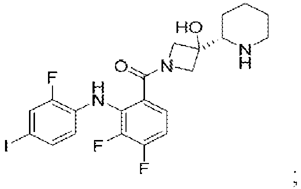 Binimetinib having the following structure: 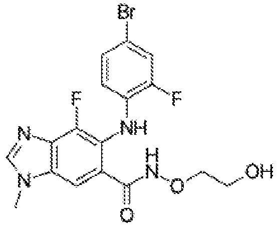 C1-1040 (also known as PD184352) having the following structure: 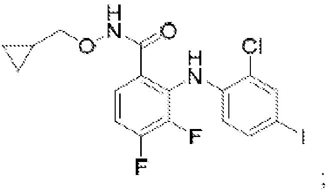 PD-325901 having the following strcuture: 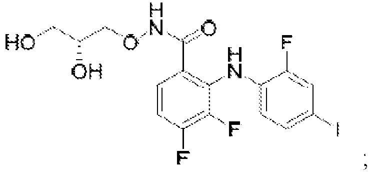 Selumetinib (also known as AZD6244) having the following structure: 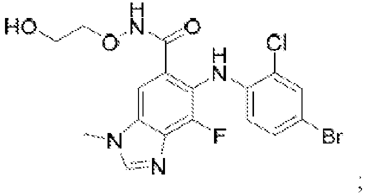 MEK162 having the following structure: 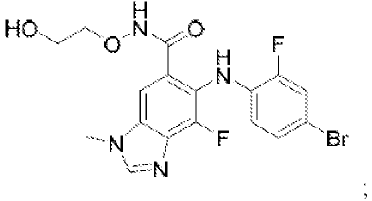 AZD8330 having the following structure: 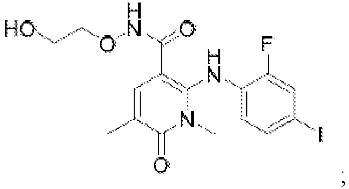 TAK-733 having the following structure: 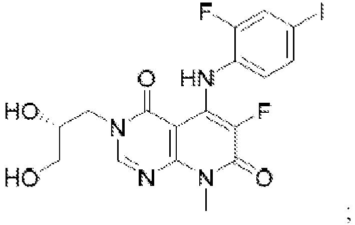 GDC-0623 having the following structure: 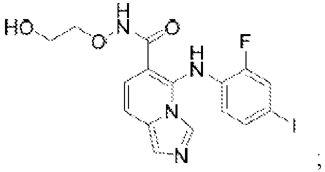 Refametinib (also known as RDEA119; BAY 869766) having the following structure: 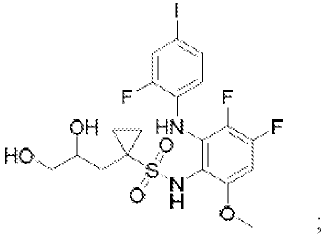 Pimasertib (also known as AS4987655) having the following structure: 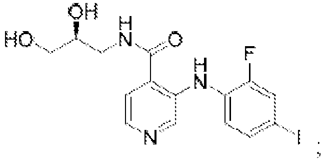 R04987655 (also known as CH4987655) having the following structure: 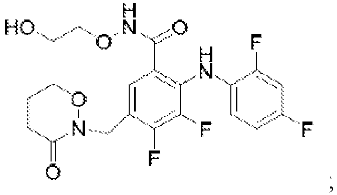 CH5126766 (VS-6766) having the following structure: 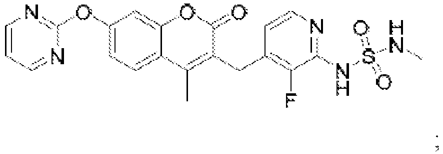 CInQ-03 having the following structure: 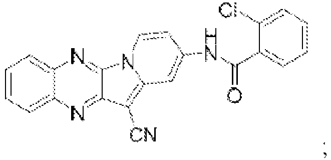 G-573 having the following structure: 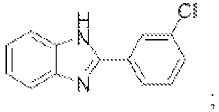 PD184161 having the following structure: 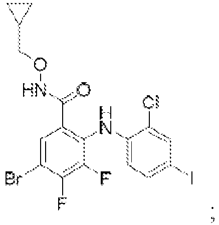 PD3 18088 having the following structure: 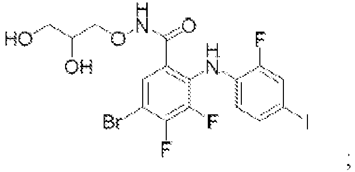 PD98059 having the following structure: 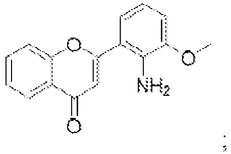 R05068760 having the following structure: 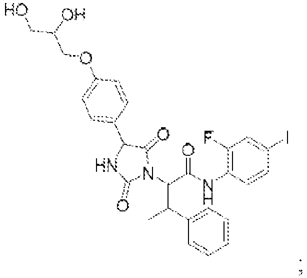 SL327 having the following structure: 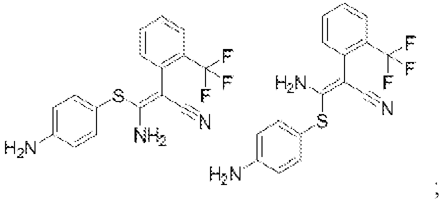 U0126 having the following structure: 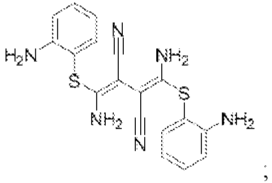 WX-554 (Wilex); and HL-085 (Shanghai Kechow Pharma).

In some embodiments, the MEK inhibitor is selected from the group consisting of trametinib, cobimetinib, binimetinib, selumetinib, PD-325901, CI-1040, CH5126766, MEK162, AZD8330, GDC-0623, refametinib, pimasertib, WX-554, HL-085, CH4987655, TAK-733, CInQ-03, G-573, PD184161, PD318088, PD98059, RO5068760, U0126, and SL327, or a pharmaceutically acceptable salt thereof.

In some embodiments, the MEK inhibitor is dosed at least once a week (e.g., once a week, twice a week, three times a week, four times a week, five times a week, or six times a week). In some embodiments, the MEK inhibitor is dosed once a week. In some embodiments, the MEK inhibitor is dosed twice a week. In some embodiments, the MEK inhibitor is dosed once daily. In some embodiments, the MEK inhibitor is dosed twice daily. In some embodiments, the MEK inhibitor is dosed at about 0.1 mg to about 100 mg, e.g., about 0.1 mg to about 50 mg, about 0.1 mg to about 10 mg, about 0.1 mg to about 5 mg, about 0.1 mg to about 4 mg, about 0.1 mg to about 3 mg, about 0.1 mg to about 2 mg, about 0.1 mg to about 1 mg, about 1 mg to about 10 mg, about 1 mg to about 20 mg, about 1 mg to about 40 mg, about 1 mg to about 60 mg, about 1 mg to about 80 mg, about 1 mg to about 100 mg, about 10 mg to about 100 mg, about 20 mg to about 100 mg, about 40 mg to about 100 mg, about 60 mg to about 100 mg, or about 80 mg to about 100 mg. In some embodiments, the MEK inhibitor is dosed at about 0.1 mg, 0.2 mg, 0.5 mg, 1 mg, 1.5 mg, 3 mg, 4 mg, 5 mg, 10 mg, 15 mg, 20 mg, 25 mg, 30 mg, 35 mg, 40 mg, 45 mg, 50 mg, 65 mg, 70 mg, 75 mg, 80 mg, 85 mg, 90 mg, 95 mg, or 100 mg. In some embodiments, the MEK inhibitor is administered orally.

In some embodiments, the MEK inhibitor is a dual RAF/MEK inhibitor. In some embodiments, the MEK inhibitor is CH5126766 or a pharmaceutically acceptable salt thereof.
In some embodiments, the dual RAF/MEK inhibitor is dosed at least once a week (e.g., once a week, twice a week, three times a week, four times a week, five times a week, or six times a week). In some embodiments, the dual RAF/MEK inhibitor is dosed once a week. In some embodiments, the dual RAF/MEK inhibitor is dosed twice a week. In some embodiments, the dual RAF/MEK inhibitor is dosed once daily. In some embodiments, the dual RAF/MEK inhibitor is dosed twice daily. In some embodiments, the dual RAF/MEK inhibitor is dosed at about 0.1 mg to about 100 mg. In some embodiments, the dual RAF/MEK inhibitor is administered orally.

In some embodiments, the dual RAF/MEK inhibitor is administered before the KRAS G12C inhibitor is administered. In some embodiments, the dual RAF/MEK inhibitor is administered after the KRAS G12C inhibitor is administered. In some embodiments, the dual RAF/MEK inhibitor is administered concurrently with the KRAS G12C inhibitor.

### Diseases and Disorders

### Abnormal Cell Growth

Abnormal cell growth, as used herein and unless otherwise indicated, refers to cell growth that is independent of normal regulatory mechanisms (e.g., loss of contact inhibition). This includes the abnormal growth of: (1) tumor cells (tumors) that proliferate, for example, by expressing a mutated tyrosine kinase or overexpression of a receptor tyrosine kinase; (2) benign and malignant cells of other proliferative diseases, for example, in which aberrant tyrosine kinase activation occurs; (3) any tumors that proliferate, for example, by receptor tyrosine kinases; (4) any tumors mat proliferate, for example, by aberrant serine/threonine kinase activation; and (5) benign and malignant cells of other proliferative diseases, for example, in which aberrant serine/threonine kinase activation occurs. Abnormal cell growth can refer to cell growth in epithelial (e.g., carcinomas, adenocarcinomas): mesenchymal (e.g., sarcomas (e.g. leiomyosarcoma. Ewing's sarcoma)); hematopoetic (e.g., lymphomas, leukemias, myelodysplasias (e.g., pre-malignant)); or other (e.g., melanoma, mesothelioma, and other tumors of unknown origin) cell.

### Neoplastic Disorders

Abnormal cell growth can refer to a neoplastic disorder. A "neoplastic disorder" is a disease or disorder characterized by cells that have the capacity for autonomous growth or replication, e.g., an abnormal state or condition characterized by proliferative cell growth. An abnormal mass of tissue as a result of abnormal cell growth or division, or a "neoplasm," can be benign, pre-malignant (carcinoma in situ) or malignant (cancer).

Exemplary neoplastic disorders include: carcinoma, sarcoma, metastatic disorders (e.g., tumors arising from prostate, colon, lung, breast and liver origin), hematopoietic neoplastic disorders, e.g., leukemias, metastatic tumors. Treatment with the compound may be in an amount effective to ameliorate at least one symptom of the neoplastic disorder, e.g., reduced cell proliferation, reduced tumor mass, etc.

### Cancer

The inventive methods of the present invention may be useful in the prevention and treatment of cancer, including for example, solid tumors, soft tissue tumors, and metastases thereof. The disclosed methods are also useful in treating non-solid cancers. Exemplary solid tumors include malignancies (e.g., sarcomas, adenocarcinomas, and carcinomas) of the various organ systems, such as those of lung, breast, lymphoid, gastrointestinal (e.g., colon), and genitourinary (e.g., renal, urothelial, or testicular tumors) tracts, pharynx, prostate, and ovary, Exemplary adenocarcinomas include colorectal cancers, renal-cell carcinoma, liver cancer (e.g.. Hepatocellular carcinoma), non-small cell carcinoma of the lung, pancreatic (e.g., metastatic pancreatic adenocarcinoma) and cancer of the small intestine.

The cancer can include mesothelioma; neurofibromatosis; e.g., neurofibromatosis type 2, neurofibromatosis type 1; renal cancer; lung cancer, non small cell lung cancer; liver cancer; thyroid cancer; ovarian; breast cancer; a nervous system tumor, schwannoma; meningioma; schwannomatosis; neuroma acoustic; adenoid cystic carcinoma; ependymoma; ependymal tumors, or any other tumor which exhibits decreased merlin expression and/or mutation, and/or deletion and/or promotor hypermethylation of the NF-2 gene. In some embodiments, the cancer is renal cancer.

The cancer can include cancers characterized as comprising cancer stem cells, cancer associated mesenchymal cells, or tumor initiating cancer cells. The cancer can include cancers that have been characterized as being enriched with cancer stem cells, cancer associated mesenchymal cells, or tumor initiating cancer cells (e.g., a tumor enriched with cells that have undergone an epithelial-to-mesenchymal transition or a metastatic tumor).

The cancer can be a primary tumor, i.e., located at the anatomical site of tumor growth initiation. The cancer can also be metastatic, i.e., appearing at least a second anatomical site other than the anatomical site of tumor growth initiation. The cancer can be a recurrent cancer, i.e., cancer that returns following treatment, and after a period of time in which the cancer was undetectable. The recurrent cancer can be anatomically located locally to the original tumor, e.g., anatomically near the original tumor; regionally to the original tumor, e.g., in a lymph node located near the original tumor; or distantly to the original tumor, e.g., anatomically in a region remote from the original tumor.

The cancer can also include for example, but is not limited to, epithelial cancers, breast, lung, pancreatic, colorectal (e.g., metastatic colorectal, e.g., metastatic KRAS mutated), prostate, head and neck, melanoma (e.g., NRAS mutated locally advanced or metastatic malignant cutaneous melanoma), acute myelogenous leukemia, and glioblastoma. Exemplary breast cancers include triple negative breast cancer, basal-like breast cancer, claudin-low breast cancer, invasive, inflammatory, metaplastic, and advanced HER-2 positive or ER-positive cancers resistant to therapy.

The cancer can also include a cancer with a KRAS G12C mutation.

The cancer can also include lung adenocarcinoma, colorectal cancer (CRC), uterine endometrioid carcinoma, bladder urothelial carcinoma, breast invasive lobular carcinoma, cervical squamous cell carcinoma, cutaneous melanoma, endocervical adenocarcinoma, hepatocellular carcinoma, pancreatic adenocarcinoma, biphasic type pleural mesothelioma, renal clear cell carcinoma, renal clear cell carcinoma, stomach adenocarcinoma, tubular stomach adenocarcinoma, uterine carcinosarcoma, or uterine malignant mixed Mullerian tumor.

Other cancers include but are not limited to, uveal melanoma, brain, abdominal, esophagus, gastrointestinal, glioma, liver, tongue, neuroblastoma, osteosarcoma, ovarian, retinoblastoma, Wilm's tumor, multiple myeloma, skin, lymphoma, blood and bone marrow cancers (e.g., advanced hematological malignancies, leukemia, e.g., acute myeloid leukemia (e.g., primary or secondary), acute lymphoblastic leukemia, acute lymphocytic leukemia, T cell leukemia, hematological malignancies, advanced myeloproliferative disorders, myelodysplastic syndrome, relapsed or refractory multiple myeloma, advanced myeloproliferative disorders), retinal, bladder, cervical, kidney, endometrial, meningioma, lymphoma, skin, uterine, lung, non small cell lung, nasopharyngeal carcinoma, neuroblastoma, solid tumor, hematologic malignancy, squamous cell carcinoma, testicular, thyroid, mesothelioma, brain vulval, sarcoma, intestine, oral, endocrine, salivary, spermatocyte seminoma, sporadic medulalry thyroid carcinoma, non-proliferating testes cells, cancers related to malignant mast cells, non-Hodgkin's lymphoma, and diffuse large B cell lymphoma.

In some embodiments, the tumor is a solid tumor. In some embodiments, the solid tumor is locally advanced or metastatic, hi some embodiments, the solid tumor is refractory (e.g., resistant) after standard therapy.

Methods described herein can reduce, ameliorate or altogether eliminate the disorder, and/or its associated symptoms, to keep it from becoming worse, to slow the rate of progression, or to minimize the rate of recurrence of the disorder once it has been initially eliminated (i.e., to avoid a relapse). A suitable dose and therapeutic regimen may vary depending upon the specific compounds, combinations, and/or pharmaceutical compositions used and the mode of delivery of the compounds, combinations, and/or pharmaceutical compositions. In some embodiments, the method increases the average length of survival, increases the average length of progression-free survival, and/or reduces the rate of recurrence, of subjects treated with the combinations described herein in a statistically significant manner.

In some embodiments, the cancer is lung cancer (e.g., non-small cell lung cancer NSCLC), e.g., KRAS mutant NSCLC; metastatic cancer), bone cancer, pancreatic cancer, skin cancer, cancer of the head or neck, cutaneous or intraocular melanoma, uterine cancer, ovarian cancer (e.g., unresectable low-grade ovarian, advanced or metastatic ovarian cancer), rectal cancer, cancer of the anal region, stomach cancer, colon cancer, breast cancer (e.g., triple-negative breast cancer (e.g., breast cancer which does not express the genes for the estrogen receptor, progesterone receiptor, and Her2/neu)), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, mesothelioma (e.g., malignant pleural mesothelioma, e.g., surgical resectable malignant pleural mesothelioma) or a combination of one or more of the foregoing cancers. In some embodiments, the cancer is metastatic. In some embodiments, the abnormal cell growth is locally recurring (e.g.. the subject has a locally recurrent disease, e.g., cancer).

### Additional Therapies

In some embodiments, the methods and compositions described herein is administered together with an additional therapy (e.g., cancer treatment). In one embodiment, a mixture of one or more compounds or pharmaceutical compositions may be administered with the combination described herein to a subject in need thereof. In yet another embodiment, one or more compounds or compositions (e.g., pharmaceutical compositions) may be administered with the combination described herein for the treatment or avoidance of various diseases, including, for example, cancer, diabetes, neurodegenerative diseases, cardiovascular disease, blood clotting, inflammation, flushing, obesity, aging, stress, etc. In various embodiments, combination therapies comprising a compound or pharmaceutical composition described herein may refer to (1) pharmaceutical compositions that comprise one or more compounds in combination with the combination described herein; and (2) co-administration of one or more compounds or pharmaceutical compositions described herein with the combination described herein, wherein the compound or pharmaceutical composition described herein have not been formulated in the same compositions. In some embodiments, the combinations described herein is administered with an additional treatment (e.g., an additional cancer treatment). In some embodiments, the additional treatment (e.g., an additional cancer treatment) can be administered simultaneously (e.g., at the same time), in the same or in separate compositions, or sequentially. Sequential administration refers to administration of one treatment before (e.g., immediately before, less than 5, 10, 15, 30, 45, 60 minutes; 1 , 2, 3, 4, 6, 8, 10, 12, 16, 20, 24, 48, 72, 96 or more hours; 4, 5, 6, 7, 8, 9 or more days; 1 , 2, 3, 4, 5, 6, 7, 8 or more weeks before) administration of an additional, e.g., secondary, treatment (e.g., a compound or therapy). The order of administration of the first and secondary compound or therapy can also be reversed.

Exemplary cancer treatments include, for example: chemotherapy, targeted therapies such as antibody therapies, immunotherapy, and hormonal therapy. Examples of each of these treatments are provided below.

### Chemotherapy

In some embodiments, a combination described herein is administered with a chemotherapy. Chemotherapy is the treatment of cancer with drugs that can destroy cancer cells. "Chemotherapy" usually refers to cytotoxic drugs which affect rapidly dividing cells in general, in contrast with targeted therapy. Chemotherapy drugs interfere with cell division in various possible ways, e.g., with the duplication of DNA or the separation of newly formed chromosomes. Most forms of chemotherapy target all rapidly dividing cells and are not specific for cancer cells, although some degree of specificity may come from the inability of many cancer cells to repair DNA damage, while normal cells generally can.

Examples of chemotherapeutic agents used in cancer therapy include, for example, antimetabolites (e.g., folic acid, purine, and pyrimidine derivatives) and alkylating agents (e.g., nitrogen mustards, nitrosoureas, platinum, alkyl sulfonates, hydrazines, triazenes, aziridines, spindle poison, cytotoxic agents, toposimerase inhibitors and others). Exemplary agents include Aclarubicin, Actinomycin, Alitretinon, Altretamine, Aminopterin, Aminolevulinic acid, Amrubicin, Amsacrine, Anagrelide, Arsenic trioxide, Asparaginase, Atrasentan, Belotecan, Bexarotene, endamustine, Bleomycin, Bortezomib, Busulfan, Camptotnecin, Capecitabine, Carboplatin, Carboquone, Carmofur, Carmustine, Celecoxib, Chlorambucil, Chlormethine, Cisplatin, Cladribine, Clofarabine, Crisantaspase, Cyclophosphamide, Cytarabine, Dacarbazine, Dactinomycin, Daunorubicin, Decitabine, Demecolcine, Docetaxel, Doxorubicin, Efaproxiral, Elesclomol, Elsamitrucin, Enocitabine, Epirubicin, Estramustine, Etoglucid, Etoposide, Floxuridine, Fludarabine, Fluorouracil (SFU), Fotemustine, Gemcitabine, Ciliadel implants, Hydroxycarbamide, Hydroxyurea, idarubicin, Ifosfamide, Irinotecan, Irofulven, Ixabepilone, Larotaxel, Leucovorin, Liposomal doxorubicin, Liposomal daunorubicin, Lonidamine, Lomustine, Lucanthone, Mannosulfan, Masoprocol, Melphalan, Mercaptopurine, Mesna, Methotrexate, Methyl aminolevulinate, Mitobronitol, Mitoguazone, Mitotane, Mitomycin, Mitoxantrone, Nedaplatin, Nimustine, Oblimersen, Omacetaxine, Ortataxel, Oxaliplatin, Paclitaxel, Pegaspargase, Pemetrexed, Pentostatin, Pirarubicin, Pixanlrone, Plicamycin, Porfimer sodium, Prednimustine, Procarbazine, Raltitrexed, Ranimustine, Rtibitecan, Sapacitabine, Semustine, Sitimagene ceradenovece, Strataplatin, Streptozocin, Talaporfm, Tegafur-uracil, Temoporfin, Temozolomide, Teniposide, Tesetaxel, Testolactone, Tetranitrate, Thiotepa, Tiazofurine, Tioguanine, Tipifarnib, Topotecan, Trabectedin, Triaziquone, Triethylenemelamine, Triplatin, Tretinoin, Treosulfan, Trofosfamide, Uramustine, Valrubicin, Verteporfin, Vinblastine, Vincristine, Vindesine, Vinflunine, Vinorelbine, Vorinostat, Zorubicin, and other cytostatic or cytotoxic agents described herein.

Because some drugs work better together than alone, two or more drugs are often given at the same time or sequentially. Often, two or more chemotherapy agents are used as combination chemotherapy. In some embodiments, the chemotherapy agents (including combination chemotherapy) can be used in combination with a combination described herein.

### Targeted Therapy

In some embodiments, a combination described herein is administered with a targeted therapy. Targeted therapy constitutes the use of agents specific for the deregulated proteins of cancer cells. Small molecule targeted therapy drugs are generally inhibitors of enzymatic domains on mutated, overexpressed, or otherwise critical proteins within the cancer cell. Prominent examples are the tyrosine kinase inhibitors such as Axitinib, Bosutinib, Cediranib, desatinib, erolotinib, imatinib, gefitinib, lapatinib, Lestaurtinib, Nilotinib, Semaxanib, Sorafenib, Sunitinib, and Vandetanib, and also cyclin-depdendent kinase inhibitors such as Alvocidib and Seliciclib. Monoclonal antibody therapy is another strategy in which the therapeutic agent is an antibody which specifically binds to a protein on the surface of the cancer cells. Examples include the anti-HER2/neu antibody trastuzumab (HERCEPTIN^{®}) typically used in breast cancer, and the anti-CD20 antibody rituximab and Tosittinioniab typically used in a variety of B-cell malignancies. Other exemplary anbitodies include Ctuximab, Panitumumab, Trastuzumab, Alemtuzumab, Bevacizumab, Edrecolornab, and Gemtuzumab. Exemplary fusion proteins include Aflitbercept and Denileukin diftitox. In some embodiments, the targeted therapy can be used in combination with a combination described herein.

Targeted therapy can also involve small peptides as "homing devices" which can bind to cell surface receptors or affected extracellular matrix surrounding the tumor. Radionuclides which are attached to these peptides (e.g., RGDs) eventually kill the cancer cell if the nuclide decay s in the vicinity of the cell. An example of such therapy includes BEXXAR^{®}.

### Immunotherapy

In some embodiments, a combination described herein is administered with an immunotherapy. Cancer immunotherapy refers to a diverse set of therapeutic strategies designed to induce the patient's own immune system to fight the tumor.

Contemporary methods for generating an immune response against tumors include intravesicular BCG immunotherapy for superficial bladder cancer, and use of interferons and other cytokines to induce an immune response in subjects with renal cell carcinoma and melanoma. Allogeneic hematopoietic stem cell transplantation can be considered a form of immunotherapy, since the donor's immune cells will often attack the tumor in a graft- versus-tumor effect. In some embodiments, the immunotherapy agents can be used in combination with a combination as described herein.

### Hormonal Therapy

In some embodiments, a combination described is administered with a hormonal therapy. The growth of some cancers can be inhibited by providing or blocking certain hormones. Common examples of honrione-sensitive tumors include certain types of breast and prostate cancers. Removing or blocking estrogen or testosterone is often an important additional treatment. In certain cancers, administration of hormone agonists, such as progestogens may be therapeutically beneficial. In some embodiments, the hormonal therapy agents can be used in combination with a combination described herein.

### Radiation Therapy

The combinations described herein can be used in combination with directed energy or particle, or radioisotope treatments, e.g., radiation therapies, e.g., radiation oncology, for the treatment of proliferative disease, e.g., cancer, e.g., cancer associated with cancer stem cells. The combinations described herein may be administered to a subject simultaneously or sequentially along with the directed energy or particle, or radioisotope treatments. For example, the combinations described herein may be administered before, during, or after the directed energy or particle, or radioisotope treatment, or a combination thereof. The directed energy or particle therapy may comprise total body irradiation, local body irradiation, or point irradiation. The directed energy or particle may originate from an accelerator, synchrotron, nuclear reaction, vacuum tube, laser, or from a radioisotope. The therapy may comprise external beam radiation therapy, teletherapy, brachy therapy, sealed source radiation therapy, systemic radioisotope therapy , or unsealed source radiotherapy. The therapy may comprise ingestion of, or placement in proximity to, a radioisotope, e.g., radioactive iodine, cobalt, cesium, potassium, bromine, fluorine, carbon. External beam radiation may comprise exposure to directed alpha particles, electrons (e.g., beta particles), protons, neutrons, positrons, or photons (e.g., radiowave, millimeter wave, microwave, infrared, visible, ultraviolet, X-ray, or gamma-ray photons). The radiation may be directed at any portion of the subject in need of treatment.

### Surgery

The combinations described herein can be used in combination with surgery, e.g., surgical exploration, intervention, biopsy, for the treatment of proliferative disease, e.g., cancer, e.g., cancer associated with cancer stem cells. The combinations described herein may be administered to a subject simultaneously or sequentially along with the surgery. For example, the combinations described herein may be administered before (preoperative), during, or after (post-operative) the surgery, or a combination thereof. The surgery may be a biopsy during which one or more cells are collected for further analysis. The biopsy may be accomplished, for example, with a scalpel, a needle, a catheter, an endoscope, a spatula, or scissors. The biopsy may be an excisional biopsy, an incisional biopsy, a core biopsy, or a needle biopsy, e.g., a needle aspiration biopsy. The surgery may involve the removal of localized tissues suspected to be or identified as being cancerous. For example, the procedure may involve the removal of a cancerous lesion, lump, polyp, or mole. The procedure may involve the removal of larger amounts of tissue, such as breast, bone, skin, fat, or muscle. The procedure may involve removal of part of, or the entirety of, an organ or node, for example, lung, throat, tongue, bladder, cervix, ovary, testicle, lymph node, liver, pancreas, brain, eye, kidney, gallbladder, stomach, colon, rectum, or intestine. In one embodiment, the cancer is breast cancer, e.g., triple negative breast cancer, and the surgery is a mastectomy or lumpectomy.

### Anti-Inflammatory Agents

A combination described herein can be administered with an anti-inflammatory agent. Anti-inflammatory agents can include, but are not limited to, non-steroidal anti-inflammatory agents (e.g., Salicylates (Aspirin (acetylsalicylic acid), Diflunisal, Salsalate), Propionic acid derivatives (Ibuprofen, Naproxen, Fenoprofen, Ketoprofen, Flurbiprofen, Oxaprozin, Loxoprofen), Acetic acid derivatives (Indomethacin, Sulindac, Etodolac, Ketorolac, Diclofenac, Nabumetone), Enolic acid (Oxicam) derivatives (Piroxicam, Meloxicam, Tenoxicam, Droxicam, Lomoxicam, Isoxicam), Fenamic acid derivatives ( Fenamates )(Mefenamic acid, Meclofenamic acid, Flufenamic acid. Tolfenamic acid). Selective COX -2 inhibitors (Coxibs) (Ceiecoxib), Sulphonanilides (Nimesulide). Steriods (e.g. Hydrocortisone (Cortisol), Cortisone acetate, Prednisone, Prednisolone, Methylprednisolone, Dexamethasone, Betamethasone, Triamcinolone, Beclometasone, Fludrocortisone acetate, Deoxycorticosterone acetate, Aldosterone).

### Analgesic Agents

Analgesics can include but are not limited to, opiates (e.g. morphine, codeine, oxycodone, hydrocodone, dihydromorphine, pethidine, buprenorphine, tramadol, venlafaxine), paracetomal and Nonsteroidal anti-inflammatory agents (e.g., Salicylates (Aspirin (acetylsalicylic acid), Diflunisal, Salsalate), Propionic acid derivatives (Ibuprofen, Naproxen, Fenoprofen, Ketoprofen, Flurbiprofen, Oxaprozin, Loxoprofen), Acetic acid derivatives (Indomethacin, Sulindac, Etodolac, Ketorolac, Diclofenac, Nabumetone), Enolic acid (Oxicam) derivatives (Piroxicam, Meloxicam, Tenoxicam, Droxicam, Lomoxicam, Isoxicam), Fenamic acid derivatives ( Fenamates )(Mefenamic acid, Meclofenamic acid, Flufenamic acid. Tolfenamic acid). Selective COX-2 inhibitors (Coxibs) (Ceiecoxib), Sulphonanilides (Nimesulide).

### Antiemetic Agents

A combination described herein can be administered with an antiemetic agent. Antiemetic agents can include, but are not limited to, 5-HT3 receptor antagonists (Dolasetron (Anzemet), Granisetron (Kytril, Sancuso), Ondansetron (Zofran), Tropisetron (Navoban), Palonosetron (Aloxi), Mirtazapine (Remeron)), Dopamine antagonists (Domperidone, Olanzapine, Droperidol, Haloperidol, Chlorpromazine, Promethazine, Prochlorperazine, Metoclopramide (Reglan), Alizapride, Prochlorperazine (Compazine, Stemzine, Buccastem, Stemetil, Phenotil), NK1 receptor antagonist (Aprepitant (Emend), Antihistamines (Cyclizine, Diphenhydramine (Benadryl), Dimenhydrinate (Gravel, Dramamine), Meclozine (Bonine, Antivert), Promethazine (Pentazine, Phenergan, Promacot), Hydroxyzine), benzodiazapines (Lorazepam, Midazolam), Anticholinergics (hyoscine), steriods (Dexamethasone).

### Combinations

The phrase, "in combination with," and the terms "co-administration," "coadministering," or "co-pi-oviditig", as used herein in the context of the administration of a compound described herein or a therapy described herein, means that two (or more) different compounds or therapies are delivered to the subject during the course of the subject's affliction with the disease or disorder (e.g., a disease or disorder as described herein, e.g., cancer), e.g., two (or more) different compounds or therapies are delivered to the subject after the subject has been diagnosed with the disease or disorder (e.g., a disease or disorder as described herein, e.g., cancer) and before the disease or disorder has been cured or eliminated or treatment has ceased for other reasons.

In some embodiments, the delivery of one compound or therapy is still occurring when the delivery of the second begins, so that there is overlap in terms of administration. This is sometimes referred to herein as "simultaneous" or "concurrent delivery." In other embodiments, the delivery of one compound or therapy ends before the delivery of the other compound or therapy begins. In some embodiments of either case, the treatment (e.g., administration of compound, composition, or therapy) is more effective because of combined administration. For example, the second compound or therapy is more effective, e.g., an equivalent effect is seen with less of the second compound or therapy, or the second compound or therapy reduces symptoms to a greater extent, than would be seen if the second compound or therapy were administered in the absence of the first compound or therapy, or the analogous situation is seen with the first compound or therapy. In some embodiments, delivery is such that the reduction in a symptom, or other parameter related to the disorder is greater than what would be observed with one compound or therapy delivered in the absence of the other. The effect of the two compounds or therapies can be partially additive, wholly additive, or great than additive (e.g., synergistic). The delivery can be such that the first compound or therapy delivered is still detectable when the second is delivered.

In some embodiments, the first compound or therapy and second compound or therapy can be administered simultaneously (e.g., at the same time), in the same or in separate compositions, or sequentially. Sequential administration refers to administration of one compound or therapy before (e.g., immediately before, less than 5, 10, 15, 30, 45, 60 minutes; 1 , 2, 3, 4, 6, 8, 10, 12, 16, 20, 24, 48, 72, 96 or more hours; 4, 5, 6, 7, 8, 9 or more days; 1 , 2, 3, 4, 5, 6, 7, 8 or more weeks before) administration of an additional, e.g., secondary, compound or therapy. The order of administration of the first and secondary compound or therapy can also be reversed.

The combinations described herein can be a first line treatment for abnormal cell growth, e.g., cancer, i.e., it is used in a patient who has not been previously administered another drug intended to treat the cancer; a second line treatment for the cancer, i.e., it is used in a subject in need thereof who has been previously administered another drug intended to treat the cancer; a third or fourth treatment for the cancer, i.e., it is used in a subject who has been previously administered two or three other drugs intended to treat the cancer.

In some embodiments, the FAK inhibitor and the KRAS G12C inhibitor are administered at amounts (e.g., doses) that result in a synergistic (e.g., therapeutic) effect.

In some embodiments, the FAK inhibitor is administered before the KRAS G12C inhibitor is administered. In some embodiments, the FAK inhibitor is administered after the KRAS G12C inhibitor is administered. In some embodiments, the FAK inhibitor is administered concurrently with the KRAS G12C inhibitor.

In some embodiments, the KRAS G12C inhibitor and the MEK inhibitor are administered at amounts (e.g., doses) that result in a synergistic (e.g., therapeutic) effect.

In some embodiments, the MEK inhibitor is administered before the KRAS G12C inhibitor is administered. In some embodiments, the MEK inhibitor is administered after the KRAS G12C inhibitor is administered. In some embodiments, the MEK inhibitor is administered concurrently with the KRAS G12C inhibitor.

In some embodiments, the FAK inhibitor, the KRAS G12C inhibitor, and the MEK inhibitor are administered at amounts (e.g., doses) that result in a synergistic (e.g., therapeutic) effect. In some embodiments, the MEK inhibitor and FAK inhibitor are administered concurrently with the KRAS G12C inhibitor.

### Administration and Dosage

The combinations of this invention may be administered orally, parenterally, topically, rectally, or via an implanted reservoir, preferably by oral administration or administration by injection. In some cases, the pH of the composition (e.g., pharmaceutical composition) may be adjusted with pharmaceutically acceptable acids, bases or buffers to enhance the stability or efficacy of the composition.

In some embodiments, the subject is administered the composition (e.g., pharmaceutical composition) orally. In some embodiments the composition (e.g., pharmaceutical composition) is be orally administered in any orally acceptable dosage form including, but not limited to, liqui-gel tablets or capsules, syrups, emulsions and aqueous suspensions. Liqui-gels may include gelatins, plasticisers, and/or opacifiers, as needed to achieve a suitable consistency and may be coated with enteric coatings that are approved for use, e.g., shellacs. Additional thickening agents, for example gums, e.g., xanthum gum, starches, e.g., corn starch, or glutens may be added to achieve a desired consistency of the composition (e.g., pharmaceutical composition) when used as an oral dosage. If desired, certain sweetening and/or flavoring and/or coloring agents may be added.

In some embodiments, the subject is administered the composition (e.g., pharmaceutical composition) in a form suitable for oral administration such as a tablet, capsule, pill, powder, sustained release formulations, solution, and suspension. The composition (e.g., pharmaceutical composition) may be in unit dosage forms suitable for single administration of precise dosages. Pharmaceutical compositions may comprise, in addition to a compound as described herein a pharmaceutically acceptable carrier, and may optionally further comprise one or more pharmaceutically acceptable excipients, such as, for example, stabilizers, diluents, binders, and lubricants. In addition, the tablet may include other medicinal or pharmaceutical agents, carriers, and or adjuvants. Exemplary pharmaceutical compositions include compressed tablets (e.g., directly compressed tablets).

Tablets are also provided comprising the active or therapeutic ingredient (e.g., compound as described herein). Tn addition to the active or therapeutic ingredients, tablets may contain a number of inert materials such as carriers. Pharmaceutically acceptable carriers can be sterile liquids, such as water and oils, including those of petroleum, animal, vegetable or synthetic origin, such as peanut oil, sesame oil and the like. Saline solutions and aqueous dextrose can also be employed as liquid earners. Oral dosage forms for use in accordance with the present invention thus may be formulated in conventional manner using one or more pharmaceutically acceptable carriers comprising excipients and auxiliaries, which facilitate processing of the active ingredients into preparations which, can be used pharmaceutically. Excipients can impart good powder flow and compression characteristics to the material being compressed. Examples of excipients are described, for example, in the Handbook of Pharmaceutical Excipients (5th edition), Edited by Raymond C Rowe, Paul J. Sheskey, and Sian C. Owen; Publisher: Pharmaceutical Press.

For oral administration, the active ingredients, e.g., the compound as described herein can be formulated readily by combining the active ingredients with pharmaceutically acceptable carriers well known in the art. Such carriers enable the active ingredients of the invention to be formulated as tablets, pills, capsules, liquids, gels, syrups, slurries, powders or granules, suspensions or solutions in water or non-aqueous media, and the like, for oral ingestion by a subject. Pharmacological preparations for oral use can be made using a solid excipient, optionally grinding the resulting mixture, and processing the mixture of granules, after adding suitable auxiliaries if desired, to obtain, for example, tablets. Suitable excipients such as diluents, binders or disintegrants may be desirable.

The dosage may vary depending upon the dosage form employed and the route of administration utilized. The exact formulation, route of administration and dosage can be chosen by the individual physician in view of the patient's condition. (See e.g., Fingl, et al., 1975, in ' he Pharmacological Basis of Therapeutics"). Lower or higher doses than those recited above may be required. Specific dosage and treatment regimens for any particular subject will depend upon a variety of factors, including the activity of the specific compound employed, the age, body weight, general health status, sex, diet, time of administration, rate of excretion, drug combination, the severity and course of the disease, condition or symptoms, the subject's disposition to the disease, condition or symptoms, and the judgment of the treating physician. A course of therapy can comprise one or more separate administrations of a compound as described herein. A course of therapy can comprise one or more cycles of a compound as described herein.

In some embodiments, a cycle, as used herein in the context of a cycle of administration of a drug, refers to a period of time for which a drug is administered to a patient. For example, if a drug is administered for a cycle of 21 days, the periodic administration, e.g., daily or twice daily, is given for 21 days. A drug can be administered for more than one cycle. Rest periods may be interposed between cycles. A rest cycle may be 1, 2, 4, 6, 8, 10, 12, 16, 20, 24 hours, 1 , 2, 3, 4, 5, 6, 7 days, or 1 , 2, 3, 4 or more weeks in length.

Oral dosage forms may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

### Examples

In order that the invention described herein may be more fully understood, the following examples are set forth. The examples described in this application are offered to illustrate the pharmaceutical compositions and methods provided herein and are not to be construed in any way as limiting their scope.

The following abbreviations may be used in the examples below: DMSO: dimethyl sulfoxide; HPCD: 2-hydroxypropyl-beta-cyclodextrin; HPMC: hydroxypropyl methylcellulose; PO: by mouth; QD: once a day; BID: twice a day; G12Ci: G12C inhibitor; FAKi: FAK inhibitor

### Example 1. Synergistic Antitumor Efficacy of the Dual RAF/MEK Inhibitor VS-6766 with KRAS G12C Inhibitors in Preclinical Solid Tumor Models

The study investigates vertical pharmacological blockade of RAS, RAF and MEK with G12C inhibitors in combination with a dual RAF/MEK inhibitor (e.g., VS-6766) +/-FAK inhibitor which the inventors have contemplated will yield superior pathway blockade and antitumor efficacy.

### Materials and Methods

### 3D proliferation assays in vitro

KRAS G12C mutant NSCLC (H2122, H358, H2030, H1373) and colorectal cancer (CRC; SW837 and SW1463) were used. Briefly, 96-well plates were coated with 50 µL of Matrigel (100%) and incubated at 37°C and 5% CO2 for 30 min in order for the Matrigel to solidify. Cells were seeded in 100 µL of 2% Matrigel containing medium. After an incubate overnight (17-22 hours) cells were treated with VS-6766 +/- G12C inhibitor +/- Defactinib for 7 days. Cell viability was measured using the cell viability CellTiter-Glo 3D assay. Combination effects were evaluated by comparing the averaged viability/ inhibition to two different null reference models derived from the single agent activities. Bliss, Loewe and Highest Single Agent synergy analysis were performed to determine synergy scores.

### Western blot

KRAS G12C mutant NSCLC cells (H2122, H358, H1373 and SW1573) were seeded in 10-cm dishes. After an incubate overnight (17~22hrs) cells were treated with 100 nM VS-6766 +/- 100 nM G12C inhibitor. 4 and 48 hours post compound treatment, cells were collected, and cell lysates prepared using RIPA buffer containing protease inhibitors. Western blot assays were run using antibodies for pMEK, MEK, pERK, ERK, p-p90RSK, p90RSK and actin.

### Xenograft tumor mouse studies

KRAS G12C mutant NSCLC H2122 and H358 tumor cells and Balb/c nude mice were used. Tumor challenge was initiated by subcutaneous inoculation of 1 x 10⁷ tumor cell suspensions into the right flank of recipient mice. Tumor sizes (mm³) and body weights were measured 3 times per week for the duration of the study. At the time of routine monitoring, the animals were checked for any effects of tumor growth and treatments on normal behavior such as mobility, food and water consumption (by looking only), and body weight gain/loss, eye/hair matting and any other abnormal effect.

For pharmacodynamic studies, once tumors reached an average volume of 200-300 mm³, mice were sorted into 4 groups (n = 5): vehicle 1 (5% DMSO, 10% HPCD in sterile water) + vehicle 2 (2% HPMC, 1% Tween 80 in sterile water), VS-6766 in vehicle 1 (0.3 mg/kg PO QD, 5 days) + vehicle 2, AMG-510 in vehicle 2 (30 mg/kg PO QD, 5 days) + vehicle 1, and VS-6766 + AMG-510.

For efficacy studies, once tumors reached an average volume of 150-200 mm³, mice were sorted into 10 groups (n = 10): vehicle 1 (5% DMSO, 10% HPCD in sterile water) + vehicle 2 (2% HPMC, 1% Tween 80 in sterile water), VS-6766 in vehicle 1 (0.3 mg/kg PO QD, 28 days) + vehicle 2, AMG-510 in vehicle 2 (30 mg/kg PO QD, 28 days) + vehicle 1, VS-6766 + AMG-510, VS-4718 (0.5% CMC-Na, 0.1% Tween 80 in sterile water) (50 mg/kg PO BID, 28 days) + vehicle 2, VS-4718 + VS-6766, VS-4718 + AMG-510, VS-4718 + AMG-510 + VS-6766, trametinib (0.3 mg/kg PO QD, 28 days) (0.5% hydroxypropylmethylcellulose and 0.2% Tween-80 in distilled water (pH 8.0)) + vehicle 2, and trametinib + AMG-510. Mice were euthanized when tumor volumes > 2,000 mm³.

### Results

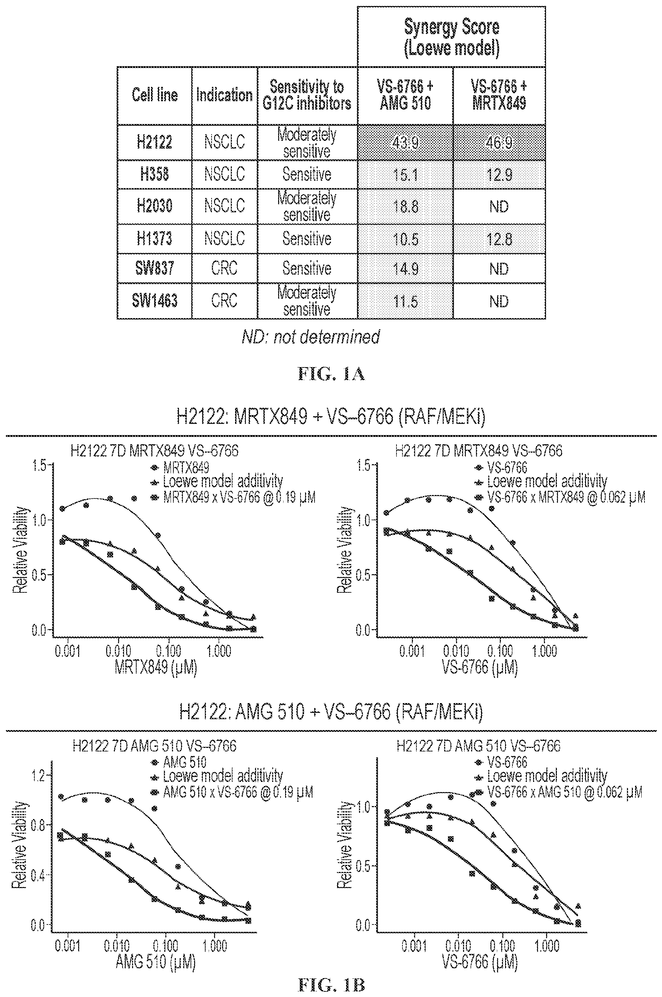

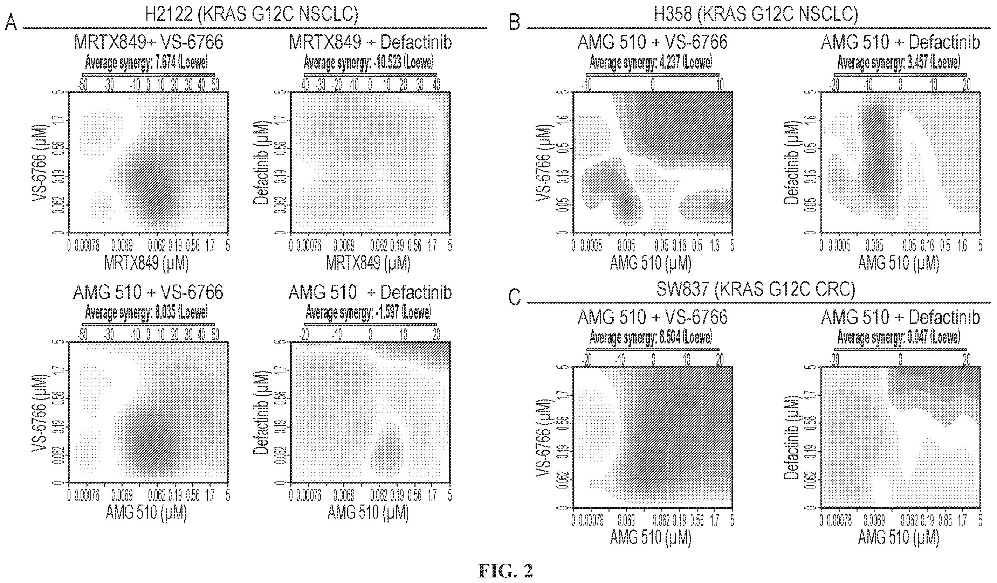

In 3D proliferation assays in vitro, VS-6766 was synergistic with both sotorasib and adagrasib in reducing viability of a panel of KRAS G12C mt NSCLC and colorectal cancer cell lines **(****FIG.s 1****, 2).** In **FIG. 1B****,** the effects of the drugs were analyzed using the Loewe Additivity model to determine if the effects of the combinations reflected the addition of the individual drug responses (Blue line (middle line): expected cancer growth inhibition if the drugs are merely additive) or synergy (Red line (lower line): deviation from blue line (middle line), indicating synergy). In **FIG. 2****,** defactinib was also shown to be synergistic with both sotorasib and adagrasib in reducing viability of a panel of KRAS G12C mt NSCLC and colorectal cancer cell lines

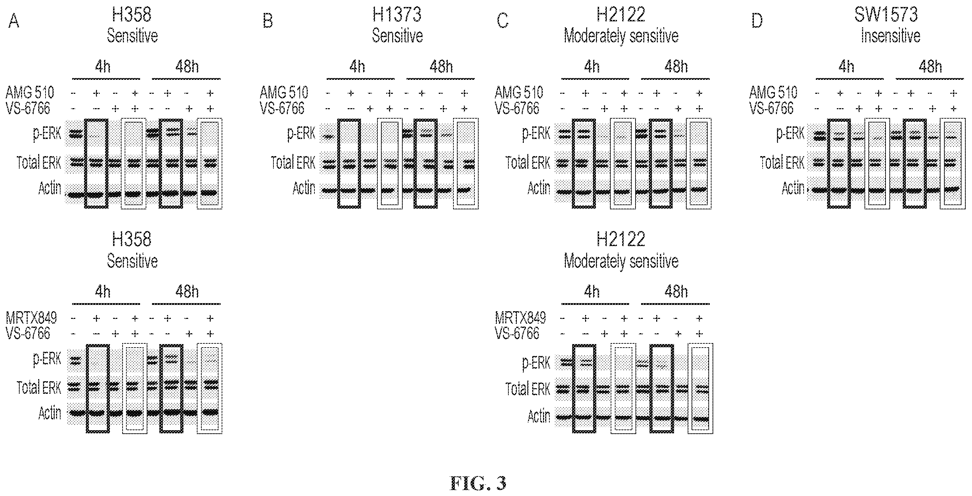

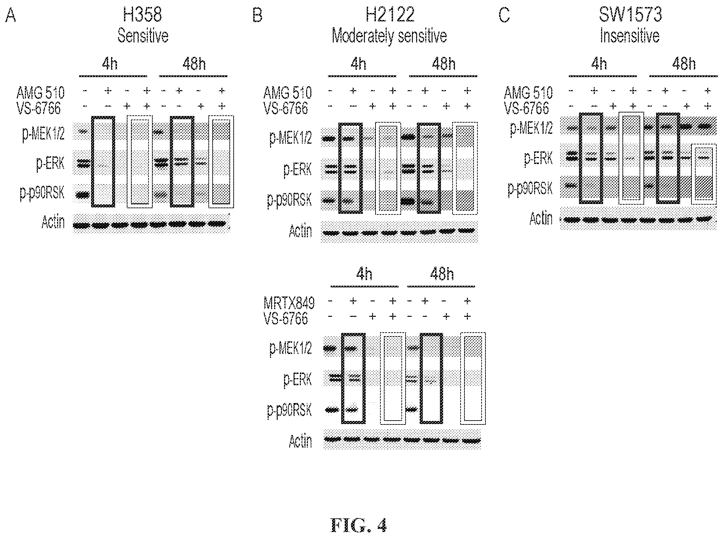

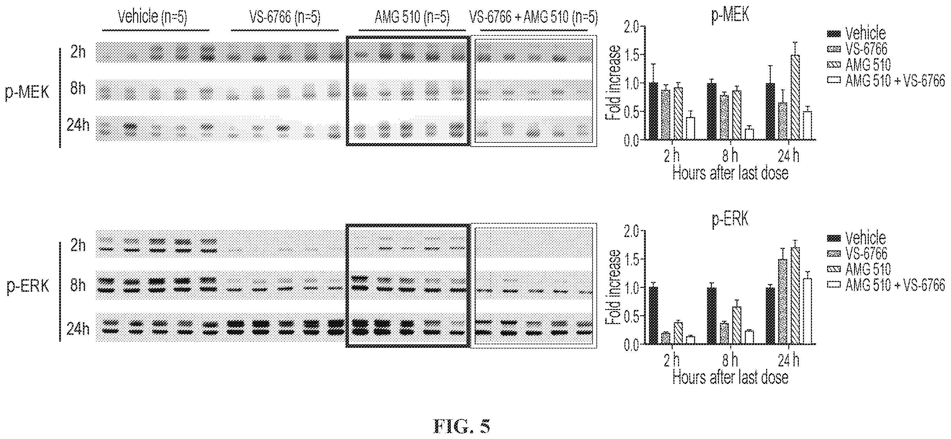

Accordingly, VS-6766 effectively suppressed RAS pathway signaling (pMEK, pERK, p-p90RSK) across KRAS-G12C NSCLC cell lines as a single agent, and the combination of VS-6766 + G12Ci showed improved depth and duration of RAS pathway signaling relative to G12Ci alone **(****FIG.s 3****, 4).** **FIG. 3** shows pERK inhibition by VS-6766 + G12C inhibitor is better than with MRTX849 or AMG-510 alone across a panel of KRAS G12C mt NSCLC cell lines. **FIG. 4** shows addition of VS-6766 to AMG-510 or MRTX849 increases depth and duration of inhibition of MEK/ERK signaling relative to G12C inhibitor alone across a panel of KRAS G12C mt NSCLC cell lines. Similarly, in the H2122 KRAS G12C NSCLC xenograft model, VS-6766 combination with sotorasib showed improved inhibition of pMEK and pERK in tumors relative to sotorasib alone **(****FIG. 5****).** VS-6766 at 0.3 mg/kg, AMG-510 at 30 mg/kg, or the combination was administrated daily for 5 days via oral gavage to mice bearing H21222 cell line xenografts (n=5/group). Tumors were harvested at 2, 8 and 24 hours following the final dose.

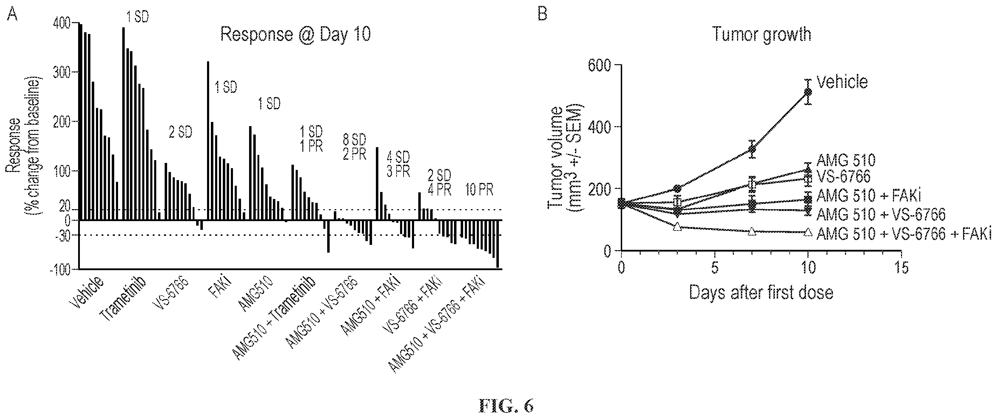

VS-6766 and FAKi potentiate AMG-510 efficacy in the H2122 KRAS G12C mutant NSCLC in vivo **(****FIG. 6****).** Combination with VS-6766 (0.3 mg/kg QD) augmented tumor growth inhibition by sotorasib (30 mg/kg QD) in the H2122 KRAS G12C mt NSCLC xenograft model, whereas trametinib (0.3 mg/kg QD) was much less effective in augmenting sotorasib efficacy. Sotorasib monotherapy induced >20% tumor regression in 0/10 mice, whereas combination of VS-6766, trametinib or a FAK inhibitor with sotorasib induced tumor regression in 5/10, 1/10 and 4/10 mice respectively following 10 days of treatment. Strikingly, triple combination of VS-6766, sotorasib and a FAK inhibitor conferred tumor reductions of ≥35% in 10/10 mice. Similar results were observed in the H358 KRAS G12C mtNSCLC model

### (FIG. 7).

### Conclusions

VS-6766 confers vertical blockade in the RAS pathway as a monotherapy. Synergy of VS-6766 + G12Ci observed across KRAS-G12C mt NSCLC & CRC cell lines. VS-6766 combination confers better ERK pathway blockade in vitro & in vivo relative to G12Ci alone. Both VS-6766 & FAKi enhance efficacy of G12Ci in H2122 & H358 xenograft models. Triple combo of G12Ci + VS-6766 + FAKi yields tumor regression in all mice. These results support the clinical evaluation of dual RAF/MEK inhibitor (e.g., VS-6766) ± a FAK inhibitor (e.g., defactinib) in combination with a G12C inhibitor for treatment of KRAS G12C mt NSCLC and CRC.

### Equivalents and Scope

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, e.g., in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.

Aspects and features of the present disclosure are set out in the following numbered clauses which contain the subject matter of the claims of the parent application as filed.
1. A method of treating a cancer in a subject in need thereof, the method comprising administering to the subject a KRAS G12C inhibitor or a pharmaceutically acceptable salt thereof in combination with a FAK inhibitor or a pharmaceutically acceptable salt thereof, thereby treating the subject.
2. The method of clause 1, wherein the FAK inhibitor is selected from the group consisting of defactinib, TAE226, BI-853520 (IN10018), GSK2256098, PF-03814735, BI-4464, VS-4718, and APG-2449, or a pharmaceutically acceptable salt thereof.
3. The method of clause 1 or 2, wherein the FAK inhibitor is defactinib or a pharmaceutically acceptable salt thereof.
4. The method of any one of clauses 1-3, wherein the KRAS G12C inhibitor is selected from the group consisting of ARS-853, ARS-1620, ARS-3248, LY3499446, AMG-510, and MRTX849, or a pharmaceutically acceptable salt thereof.
5. The method of any one of clauses 1-4, wherein the KRAS G12C inhibitor is AMG-510 or a pharmaceutically acceptable salt thereof.
6. The method of any one of clauses 1-4, wherein the KRAS G12C inhibitor is MRTX849 or a pharmaceutically acceptable salt thereof.
7. The method of any one of clauses 1-6, wherein the method further comprises administering a MEK inhibitor.
8. The method of clause 7, wherein the MEK inhibitor is selected from the group consisting of trametinib, cobimetinib, binimetinib, selumetinib, PD-325901, CI-1040, CH5126766, MEK162, AZD8330, GDC-0623, refametinib, pimasertib, WX-554, HL-085, CH4987655, TAK-733, CInQ-03, G-573, PD184161, PD318088, PD98059, RO5068760, U0126, and SL327, or a pharmaceutically acceptable salt thereof.
9. The method of clause 7, wherein the MEK inhibitor is a dual RAF/MEK inhibitor.
10. The method of any one of clauses 7-9, wherein the MEK inhibitor is CH5126766 or a pharmaceutically acceptable salt thereof.
11. The method of any one of clauses 1-10, wherein the FAK inhibitor (e.g., defactinib) is dosed twice daily.
12. The method of any one of clauses 1-10, wherein the FAK inhibitor (e.g., defactinib) is dosed once daily.
13. The method of any one of clauses 1-12, wherein the FAK inhibitor (e.g., defactinib) is dosed at about 100 mg to about 1000 mg.
14. The method of any one of clauses 1-13, wherein the FAK inhibitor (e.g., defactinib) is dosed at about 200 mg to about 400 mg.
15. The method of any one of clauses 1-14, wherein the FAK inhibitor (e.g., defactinib) is administered orally.
16. The method of any one of clauses 1-15, wherein the KRAS G12C inhibitor is dosed at about 100 mg to about 2000 mg.
17. The method of any one of clauses 1-16, wherein the KRAS G12C inhibitor is administered once daily.
18. The method of any one of clauses 1-16, wherein the KRAS G12C inhibitor is administered twice daily.
19. The method of any one of clauses 1-18, wherein the KRAS G12C inhibitor is administered orally.
20. The method of any one of clauses 1-19, wherein the FAK inhibitor is administered before the KRAS G12C inhibitor is administered.
21. The method of any one of clauses 1-19, wherein the FAK inhibitor is administered after the KRAS G12C inhibitor is administered.
22. The method of any one of clauses 1-19, wherein the FAK inhibitor is administered concurrently with the KRAS G12C inhibitor.
23. The method of any one of clauses 7-22, wherein the MEK inhibitor is dosed at least once a week (e.g., once a week, twice a week, three times a week, four times a week, five times a week, or six times a week).
24. The method of any one of clauses 7-23, wherein the MEK inhibitor is dosed once a week.
25. The method of any one of clauses 7-23, wherein the MEK inhibitor is dosed twice a week.
26. The method of any one of clauses 7-22, wherein the MEK inhibitor is dosed once daily.
27. The method of any one of clauses 7-22, wherein the MEK inhibitor is dosed twice daily.
28. The method of any one of clauses 7-27, wherein the MEK inhibitor is dosed at about 0.1 mg to about 100 mg.
29. The method of any one of clauses 7-28, wherein the MEK inhibitor is administered orally.
30. The method of any one of clauses 7-29, wherein the FAK inhibitor is defactinib or a pharmaceutically acceptable salt thereof, the KRAS G12C inhibitor is MRTX849 or a pharmaceutically acceptable salt thereof, and the MEK inhibitor is CH5126766 or a pharmaceutically acceptable salt thereof.
31. The method of any one of clauses 7-29, wherein the FAK inhibitor is defactinib or a pharmaceutically acceptable salt thereof, the KRAS G12C inhibitor is AMG-510 or a pharmaceutically acceptable salt thereof, and the MEK inhibitor is CH5126766 or a pharmaceutically acceptable salt thereof.
32. A method of treating a cancer in a subject in need thereof, the method comprising administering to the subject a KRAS G12C inhibitor or a pharmaceutically acceptable salt thereof in combination with a dual RAF/MEK inhibitor or a pharmaceutically acceptable salt thereof, thereby treating the subject.
33. The method of clause 32, wherein the KRAS G12C inhibitor is selected from the group consisting of ARS-853, ARS-1620, ARS-3248, LY3499446, AMG-510, and MRTX849, or a pharmaceutically acceptable salt thereof.
34. The method of clause 32 or 33, wherein the KRAS G12C inhibitor is AMG-510 or a pharmaceutically acceptable salt thereof.
35. The method of clause 32 or 33, wherein the KRAS G12C inhibitor is MRTX849 or a pharmaceutically acceptable salt thereof.
36. The method of any one of clauses 32-35, wherein the dual RAF/MEK inhibitor is CH5126766 or a pharmaceutically acceptable salt thereof.
37. The method of any one of clauses 32-36, wherein the KRAS G12C inhibitor is dosed at about 100 mg to about 2000 mg.
38. The method of any one of clauses 32-37, wherein the KRAS G12C inhibitor is administered once daily.
39. The method of any one of clauses 32-37, wherein the KRAS G12C inhibitor is administered twice daily.
40. The method of any one of clauses 32-39, wherein the KRAS G12C inhibitor is administered orally.
41. The method of any one of clauses 32-40, wherein the dual RAF/MEK inhibitor is dosed at least once a week (e.g., once a week, twice a week, three times a week, four times a week, five times a week, or six times a week).
42. The method of any one of clauses 32-41, wherein the dual RAF/MEK inhibitor is dosed once a week.
43. The method of any one of clauses 32-41, wherein the dual RAF/MEK inhibitor is dosed twice a week.
44. The method of any one of clauses 32-41, wherein the dual RAF/MEK inhibitor is dosed once daily.
45. The method of any one of clauses 32-41, wherein the dual RAF/MEK inhibitor is dosed twice daily.
46. The method of any one of clauses 32-45, wherein the dual RAF/MEK inhibitor is dosed at about 0.1 mg to about 100 mg.
47. The method of any one of clauses 32-46, wherein the dual RAF/MEK inhibitor is administered orally.
48. The method of any one of clauses 32-47, wherein the dual RAF/MEK inhibitor is administered before the KRAS G12C inhibitor is administered.
49. The method of any one of clauses 32-47, wherein the dual RAF/MEK inhibitor is administered after the KRAS G12C inhibitor is administered.
50. The method of any one of clauses 32-47, wherein the dual RAF/MEK inhibitor is administered concurrently with the KRAS G12C inhibitor.
51. The method of any one of clauses 1-50, wherein the cancer is a cancer with a KRAS G12C mutation.
52. The method of any one of clauses 1-51, wherein the cancer is lung adenocarcinoma, nonsmall cell lung carcinoma, colorectal cancer (CRC), uterine endometrioid carcinoma, bladder urothelial carcinoma, breast invasive lobular carcinoma, cervical squamous cell carcinoma, cutaneous melanoma, endocervical adenocarcinoma, hepatocellular carcinoma, pancreatic adenocarcinoma, biphasic type pleural mesothelioma, renal clear cell carcinoma, renal clear cell carcinoma, stomach adenocarcinoma, tubular stomach adenocarcinoma, uterine carcinosarcoma, or uterine malignant mixed Mullerian tumor.

## Claims

1. A combination of a KRAS G12C inhibitor or a pharmaceutically acceptable salt thereof and a FAK inhibitor or a pharmaceutically acceptable salt thereof, for use in a method of treating a cancer in a subject in need thereof.

2. The combination for use according to claim 1, wherein the FAK inhibitor is selected from the group consisting of defactinib, TAE226, BI-853520 (IN10018), GSK2256098, PF-03814735, BI-4464, VS-4718, and APG-2449, or a pharmaceutically acceptable salt thereof.

3. The combination for use according to claims 1 or 2, wherein the FAK inhibitor is defactinib or a pharmaceutically acceptable salt thereof.

4. The combination for use according to any one of claims 1-3, wherein the KRAS G12C inhibitor is selected from the group consisting of ARS-853, ARS-1620, ARS-3248, LY3499446, AMG-510, and MRTX849, or a pharmaceutically acceptable salt thereof.

5. The combination for use according to any one of claims 1-4, wherein the KRAS G12C inhibitor is AMG-510 or a pharmaceutically acceptable salt thereof.

6. The combination for use according to any one of claims 1-4, wherein the KRAS G12C inhibitor is MRTX849 or a pharmaceutically acceptable salt thereof.

7. The combination for use according to any one of claims 1-6, wherein the method further comprises administering a MEK inhibitor.

8. The combination for use according to claim 7, wherein the MEK inhibitor is selected from the group consisting of trametinib, cobimetinib, binimetinib, selumetinib, PD-325901, CI-1040, CH5126766, MEK162, AZD8330, GDC-0623, refametinib, pimasertib, WX-554, HL-085, CH4987655, TAK-733, CInQ-03, G-573, PD184161, PD318088, PD98059, RO5068760, U0126, and SL327, or a pharmaceutically acceptable salt thereof.

9. The combination for use according to claim 7, wherein the MEK inhibitor is a dual RAF/MEK inhibitor.

10. The combination for use according to any one of claims 7-9, wherein the MEK inhibitor is CH5126766 or a pharmaceutically acceptable salt thereof.

11. The combination for use according to any one of claims 1-10, wherein the FAK inhibitor (e.g., defactinib) is dosed twice daily.

12. The combination for use according to any one of claims 1-10, wherein the FAK inhibitor (e.g., defactinib) is dosed once daily.

13. The combination for use according to any one of claims 1-12, wherein the FAK inhibitor (e.g., defactinib) is dosed at about 100 mg to about 1000 mg.

14. The combination for use according to any one of claims 1-13, wherein the FAK inhibitor (e.g., defactinib) is dosed at about 200 mg to about 400 mg.

15. The combination for use according to any one of claims 1-14, wherein the FAK inhibitor (e.g., defactinib) is administered orally.
